# EUROPEAN PATENT APPLICATION

(11) **EP 4 578 481 A1**
(43) Date of publication of application: **02.07.2025**
(21) Application number: 24222722.1
(22) Date of filing: 23.12.2024
(51) Int. Cl.: A61M 25/01, A61B 18/14

(54) **DEFLECTABLE MEDICAL PROBE WITH ANCHOR MEMBER**

(30) Priority: 27.12.2023 US 202318397675; 06.02.2024 US 202418433765
(71) Applicant: Biosense Webster (Israel) Ltd., 2066717 Yokneam (IL)
(72) Inventor: KLEIN, Sarah, Irvine, 92618 (US); JAVIER, Carl, Irvine, 92618 (US)
(74) Representative: Carpmaels & Ransford LLP

(57) **Abstract**

The disclosed technology includes a medical probe that includes an elongated tube (131) extending along a longitudinal axis from a proximal end to a distal end, an actuator member extending along the elongated tube (131) from approximately the proximal end to approximately the distal end, an anchor member (130) attached to the actuator member near the distal end of the elongated tube (131), and a mechanical barrier (500) disposed between the anchor member (130) and the elongated tube (131). The mechanical barrier (500) can include a lumen (120) extending through the mechanical barrier (500) along a lumen axis. The lumen (120) can be configured to at least partially receive the anchor member (130).

## Description

### CROSS-REFERENCE TO RELATED APPLICATIONS

The present application is a continuation-in-part application of U.S. Patent Application Number 18/397,675, filed December 27, 2023 (Attorney Docket #: 253757.000415 - BIO6878USNP1), the entire contents of each of which is incorporated herein by reference in its entirety as if fully set forth herein.

### FIELD

The present invention relates generally to medical devices, and in particular, devices for catheters that can cause the distal end of the catheter to deflect away from a longitudinal axis.

### BACKGROUND

Catheter devices are commonly used in medical procedures when medical devices must reach organs, tissue, or other difficult-to-reach portions of a patient's body. Typically, catheters include a flexible tube or shaft that is inserted through a patient's vascular system or other regions and navigated to the target location. To help guide the catheter to the target location, catheters often include a flexible distal tip that can be articulated to help navigate the catheter through the patient's vascular system. Often, the articulation systems include a puller wire or a push rod that is anchored near a distal end of the flexible tube or shaft and is connected to an actuator on a handle operated by a medical professional. When the medical professional actuates the actuator to pull the puller wire proximally or push the push rod distally, the flexible distal tip articulates in a predetermined direction (i.e., bends away from the longitudinal axis of the flexible tube).

Some existing catheter articulation systems include anchor members that are configured to connect the distal end of the flexible tube to either the puller wire or the push rod. Some existing anchor members, however, may cut into or otherwise damage the flexible tube if too much tension or pressure is applied to the puller wire or push rod. When this happens, the puller wire or push rod may become ineffective to cause the flexible distal tip to articulate.

Accordingly, there is a need in the art for articulation systems that are configured to help prevent the likelihood that the anchor member damages the flexible tube. These and other issues can be addressed by the technology disclosed herein.

### SUMMARY

The disclosed technology includes a medical probe that includes an elongated tube extending along a longitudinal axis from a proximal end to a distal end, an actuator member extending along the elongated tube from approximately the proximal end to approximately the distal end, an anchor member attached to the actuator member near the distal end of the elongated tube, and a mechanical barrier disposed between the anchor member and the elongated tube. The mechanical barrier can include a lumen extending through the mechanical barrier along a lumen axis. The lumen can be configured to at least partially receive the anchor member.

The disclosed technology can further include a medical device including an elongated tube extending along a longitudinal axis from a proximal end to a distal end, an actuator member extending along the elongated tube, and an anchor member attached to the actuator member near the distal end of the elongated tube. The actuator member and anchor member can be configured to cause a deflectable tip of the elongated tube to deflect radially outward from the longitudinal axis when actuated. The medical device can further include a mechanical barrier disposed between the anchor member and the elongated tube. The mechanical barrier can be configured to prevent the anchor member from damaging the elongated tube.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is a schematic pictorial illustration of a medical system including a medical probe, in accordance with an embodiment of the disclosed technology;
FIG. 2 is a schematic pictorial illustration of a mechanical barrier for a medical probe, in accordance with the disclosed technology;
FIG. 3A is a front perspective view of a medical probe comprising of an elongated tube, an anchor member, and a mechanical barrier in a first position, in accordance with the disclosed technology;
FIG. 3B is a front perspective view of a medical probe comprising of an elongated tube, an anchor member, and a mechanical barrier in a second position, in accordance with the disclosed technology;
FIG. 4 is a top view of a medical probe comprising of an elongated tube, an anchor member, and a mechanical barrier in the first position, in accordance with the disclosed technology;
FIG. 5 is a front perspective view of a medical probe comprising of an elongated tube, an anchor member, and an alternative mechanical barrier, in accordance with the disclosed technology;
FIG. 6 is a perspective view of another example mechanical barrier, in accordance with the disclosed technology;
FIG. 7 is a perspective view of yet another example mechanical barrier and a fastener, in accordance with the disclosed technology;
FIG. 8A is a side perspective view of a medical probe comprising an elongated tube, an anchor member, a mechanical barrier, and a fastener, in accordance with the disclosed technology;
FIG. 8B is a side perspective view of the medical probe shown in FIG. 8A with the fastener assembled with the mechanical barrier, in accordance with the disclosed technology;
FIG. 8C is a top view of the medical probe shown in FIG. 8A with the fastener assembled with the mechanical barrier, in accordance with the disclosed technology;
FIGs. 9A and 9B are flowcharts illustrating a methods of manufacture for a medical probe, in accordance with the disclosed technology;
FIGs. 10A and 10B are flowcharts illustrating alternative methods of manufacture for a medical probe, in accordance with the disclosed technology; and
FIGs. 11A and 11B are flowcharts illustrating yet other methods of manufacture for a medical probe, in accordance with the disclosed technology.

### DETAILED DESCRIPTION

The disclosed technology includes devices for catheter articulation systems that can help to prevent anchor members from damaging the flexible tube. Furthermore, the devices described herein can help to better secure the anchor to the distal end of the flexible tube and can provide leverage to the distal end of the flexible tube to help cause the distal end of the flexible tube to deflect away from the longitudinal axis when the puller wire or push rod is actuated. The disclosed technology includes various designs for mechanical barriers that can be disposed between the anchor and the flexible tube to help protect the flexible tube. In this way, the disclosed technology can be configured for use with existing catheter systems and serves as a simple and cost-effective way to prevent the anchor from damaging the flexible tube.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives, and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician", "operator", or "medical professional" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing energy delivered to the tissue. These techniques can include ablation techniques such as irreversible electroporation (IRE), radio frequency (RF) ablation, and cryoablation.

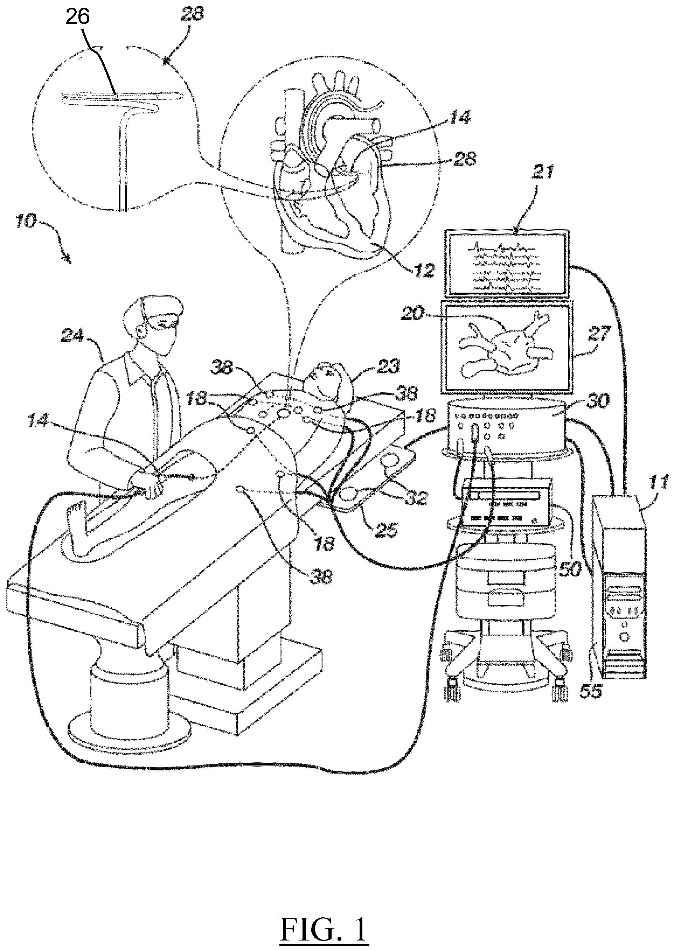

Reference is made to FIG. 1 showing an example catheter-based electrophysiology mapping and ablation system 10. System 10 includes one or more catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The one or more catheters may include catheters dedicated for sensing Intracardiac Electrogram (IEGM) signals, catheters dedicated for ablating and/or catheters dedicated for both sensing and ablating. For ablation, physician 24 brings end effector 28 comprising ablation electrodes to a target site for ablating. If the end effector 28 is alternatively or additionally configured for mapping of electrophysiological signals (e.g., IEGM signals), physician 24 similarly brings the end effector 28 into contact with the target site (e.g., the heart wall) for sensing IEGM signals at the target site in heart 12.

Catheter 14 is an exemplary catheter that includes an end effector 28 comprising one and preferably multiple electrodes 26 optionally distributed over an expandable assembly and a distal tip of end effector 28 and configured to detect electro-physiological signals and/or deliver ablative energy to tissue. Catheter 14 is shown as being a lasso catheter in FIG. 1, however, it will be appreciated that the disclosed technology can be used with other types of catheters, including, but not limited to: basket catheters, balloon catheters, planar catheters, focal catheters, and delivery sheaths.

Catheter 14 may additionally include a magnetic-based position sensor embedded in or near end effector 28 for tracking position and orientation of end effector 28. The end effector 28 can further include one or more impedance-based electrodes disposed in or near end effector 28 for tracking position and orientation of end effector 28.

Magnetic-based position sensor may be operated together with a location pad 25 including a plurality of magnetic coils 32 configured to generate magnetic fields in a predefined working volume. Real time position of end effector 28 of catheter 14 may be tracked based on magnetic fields generated with location pad 25 and sensed by magnetic-based position sensor 29. The magnetic-based position sensor can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as tracking of impedance-based electrodes. For impedance-based tracking, electrical current is directed toward impedance-based electrodes and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which is incorporated herein by reference as if set forth fully herein.

A recorder 11 displays electrograms 21 captured with body surface ECG electrodes 18 and intracardiac electrograms (IEGM) captured with electrodes of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes disposed on the end effector and configured for delivering ablative energy to tissue. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTOTM 3 System, available from Biosense Webster, Inc., 31 Technology Drive, Suite 200, Irvine, CA 92618, USA.

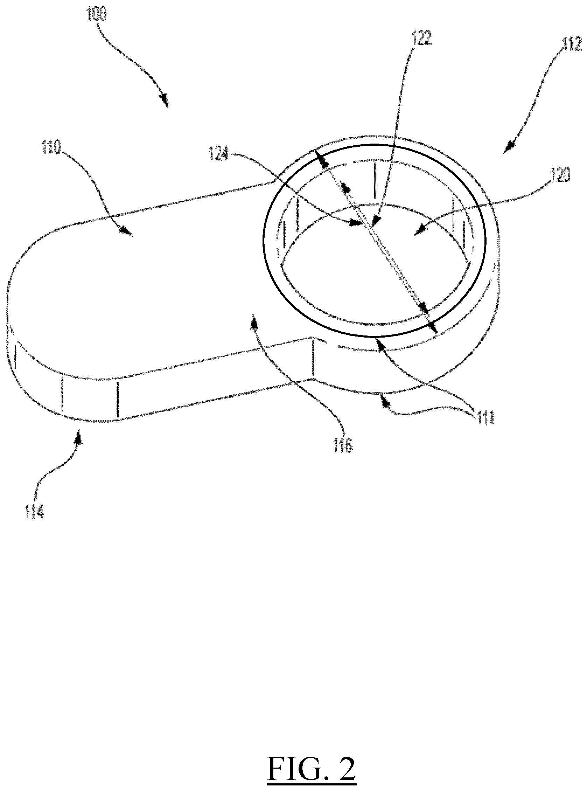

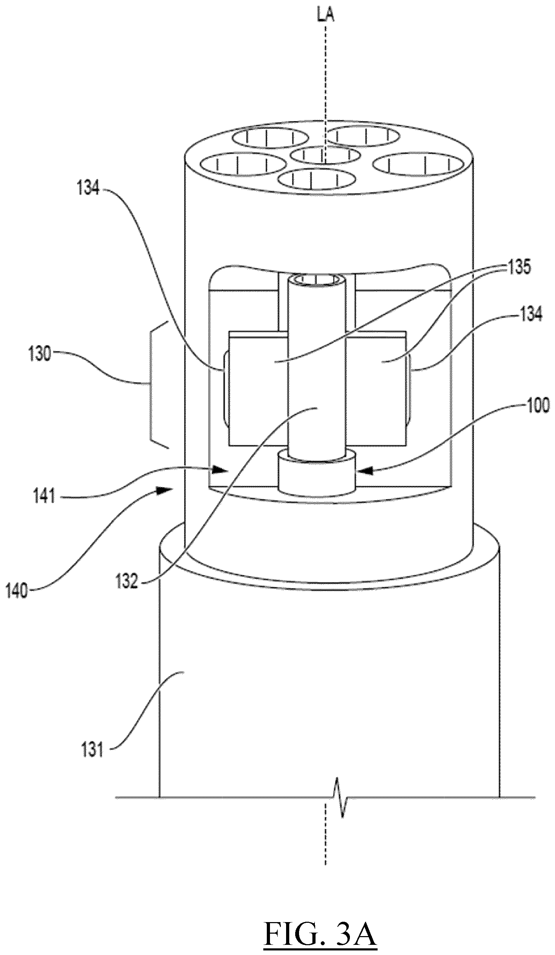

FIG. 2 is a schematic pictorial illustration of a mechanical barrier 100 for a medical probe (e.g., catheter 14), in accordance with the disclosed technology. As previously explained, existing catheters often include a pull wire or push rod attached to an anchor at a distal end of the catheter. If a medical professional pulls or pushes too hard on the pull wire or push rod, respectively, the anchor may apply too much force to the elongated tube and damage the elongated tube. Accordingly, as will become apparent throughout this disclosure, the mechanical barrier 100 can be assembled with anchor (see 130 in FIG. 3A,) at a distal end (deflectable tip 140) of the elongated tube (see 131 in FIG. 3A) of the catheter 14 and help to prevent the anchor from damaging the elongated tube.

The mechanical barrier 100 can include a body 110 extending from a first end 112 to a second end 114. The body 110 can be made of a suitable biocompatible material having a hardness that is greater than the elongated tube 131. The body 110 can define a lumen 120 extending through the body 110 near the first end 112 and an arm 116 extending outwardly toward the second end 114. The lumen 120 can have an inner diameter 122 and the body 110 form a circular end near the first end 112 defining an outer diameter 124. The outer diameter 124 can be greater than the inner diameter 122. The body 110 can further include rounded edges 111 to reduce the likelihood that the mechanical barrier 100 will damage the elongated tube 131. The body 110 can be sized as shown in FIG. 2, or the body 110 can be enlarged on one side, taper from one side to another, be thicker on one side, and or any other shape suitable for the particular application.

FIG. 3A is a front perspective view of a medical probe (e.g., catheter 14) comprising an elongated tube 131, an anchor member 130, and a mechanical barrier 100 in a first position, in accordance with the disclosed technology. The portion of the medical probe shown in FIG. 3A comprises an elongated tube 131 extending along a longitudinal axis LA and comprising a deflectable tip 140. For example, the portion of the medical probe shown in FIG. 3A can be a deflectable tip 140 near a distal end of the elongated tube 131 that can be configured to articulate for navigation of the catheter 14 to a target area within a patient's body. The deflectable tip 140 can further include a recess 141 formed into an edge of the deflectable tip 140 configured to receive the anchor member 130 such that the anchor member 130 can better fit within the catheter 14.

The anchor member 130 can be configured to attach to a pull wire or a push rod (not shown). For example, the anchor member 130 can comprise a ferrule 132 that can be crimped, soldered, adhered, or otherwise attached to the pull wire or push rod such that when a medical professional actuates the pull wire or push rod, the force applied to the pull wire or push rod will be distributed to the anchor member 130 and, consequently, to the deflectable tip 140. The anchor member 130 can further include one or more extensions 134 extending outwardly from the ferrule 132 to prevent the anchor member 130 from being pulled back through a lumen of the elongated tube 131. The anchor member 130 can further include one or more pads 135 attached to the extensions 134 to help prevent further damage by the anchor member 130 as is it pulled proximally or pushed distally.

As shown in FIG. 3A, the mechanical barrier 100 can be disposed on a proximal side of the anchor member 130 such that when a medical professional pulls on a pull wire, the anchor member 130 will contact the mechanical barrier 100 and the mechanical barrier 100 will help prevent the anchor member 130 from damaging the elongated tube 131. The anchor member 130 can be configured to at least partially extend into the lumen 120 of the mechanical barrier 100. In some examples, the ferrule 132 can extend through the lumen 120 such that the mechanical barrier 100 is prevented from prevented from being removed from the anchor member 130 and the elongated tube 131. The anchor member 130, however, can be permitted to freely rotate within lumen 120.

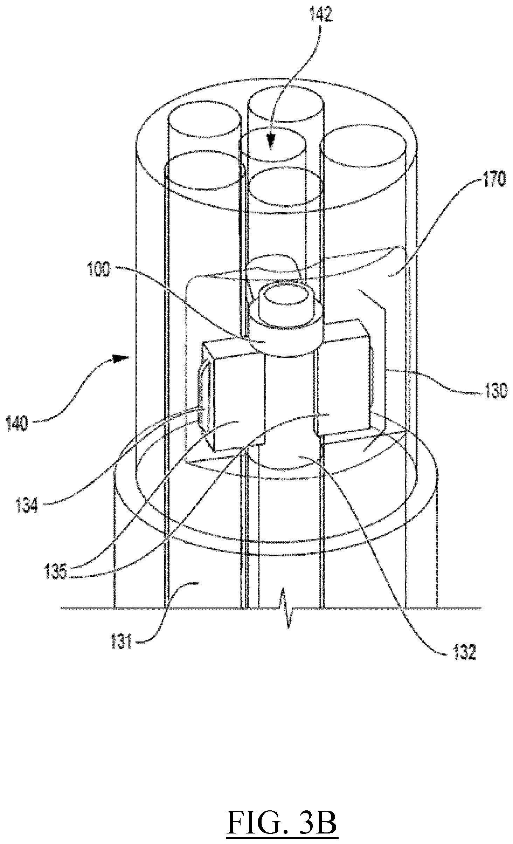

FIG. 3B is a front perspective view of a medical probe comprising of an elongated tube 131, an anchor member 130, and a mechanical barrier 100 in a second position, in accordance with the disclosed technology. In this example, the mechanical barrier 100 can be positioned on a distal end of the anchor member 130 such that when a medical professional pushes a push rod (not shown) that is attached to the anchor, the mechanical barrier 100 can help to prevent the anchor member 130 from damaging the elongated tube 131 on a distal end of the elongated tube 131. All other features shown in FIG. 3B can be similar to the features shown and described in relation to FIG. 3A.

The elongated tube 131, in the example shown in FIG. 3B, is shown as semi-transparent to illustrate the arm 116 of the mechanical barrier 100 extending at least partially into a central lumen 142 of the elongated tube 131. For example, the arm 116 can extend through a slot formed into the elongated tube 131 that extends from out an outer portion of the elongated tube 131 to an inner portion of the elongated tube 131 (e.g., the central lumen 142). The arm 116 can help to distribute a force applied to the anchor member 130 by providing a moment arm toward a center of the elongated tube 131, thereby enabling better deflection of the deflectable tip 140 as compared to existing systems.

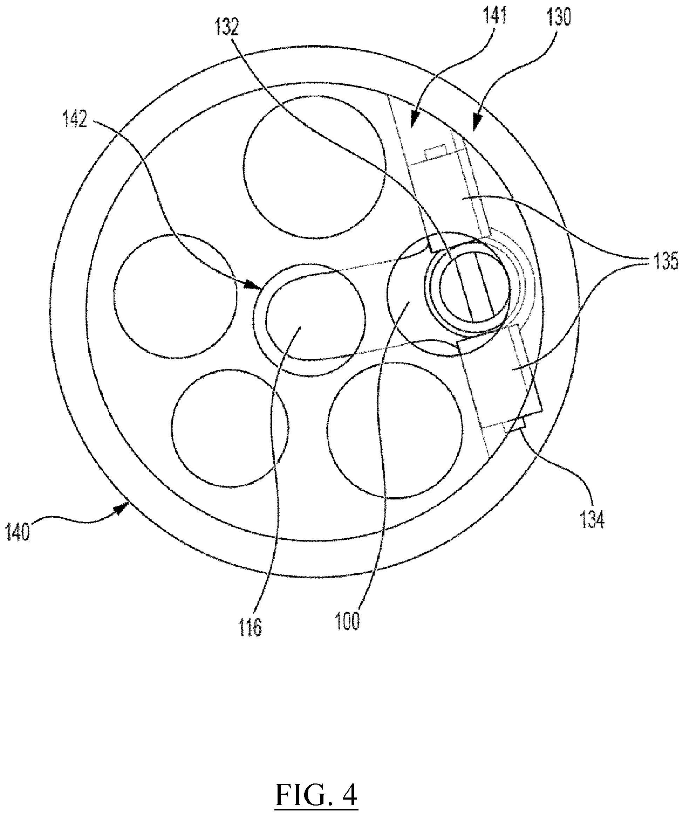

FIG. 4 is a top view of a medical probe comprising of an elongated tube 131, an anchor member 130, and a mechanical barrier 100 in the first position, in accordance with the disclosed technology. As shown, the arm 116 can extend substantially all the way toward a center of the deflectable tip 140 into a central lumen 142. The elongated tube 131 can include additional lumens configured for electrical wiring, irrigation fluid, or other components. In some examples, the arm 115 can extend into one or more lumens in addition to, or in place of, the central lumen 142. FIG. 4 also shows an alternate view of the recess 141 formed into the deflectable tip 140. As shown, the recess 141 can be sized to receive the anchor member 130 such that the anchor member 130 can fit within an outer edge of the deflectable tip 140 so that an end effector can be attached to the deflectable tip. The end effector, for example, can be an end effector configured for ablation and/or mapping of tissue that is attached to the deflectable tip 140. In some examples, the mechanical barrier 100 can be attached to components extending through the central lumen 142 (or other lumens formed in the elongated tube 131) such as components of an end effector, electrical components, or irrigation components to help provide additional support for such components.

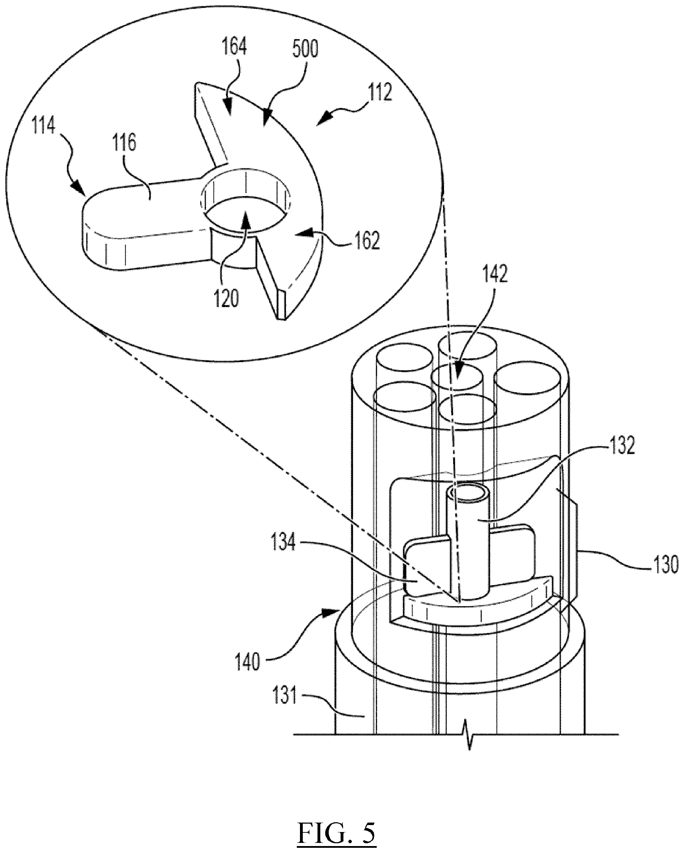

FIG. 5 is a front perspective view of a medical probe comprising of an elongated tube 131, an anchor member 130, and an alternative mechanical barrier 500, in accordance with the disclosed technology. As shown, the mechanical barrier 500 can be substantially similar to the mechanical barrier 100 except that the mechanical barrier 500 can further include a first wing 162 and a second wing 164 extending outwardly from the body 110 near the first end 112 on either side of the lumen 120. The first wing 162 and the second wing 164 can be configured to fit within the recess 141 and contact the elongated tube 131. The first wing 162 and the second wing 164 can each extend outwardly from the body 110 in a semi-circular shape. As will be appreciated, the first wing 162 and the second wing 164 can be configured to help distribute a force applied to the elongated tube 131 by the anchor member 130 via the mechanical barrier 500 by increasing the area the force from the anchor member 130 is distributed across. It will be appreciated, that the first wing 162 and the second wing 164 can comprises different shapes and sizes and can be configured as a single enlarged component extending from the body 110. The first wing 162 and the second wing 164 can be approximately symmetrical with respect to each other or the first wing 162 and the second wing 164 can be differently shaped, sized, or otherwise arranged depending on the particular configuration.

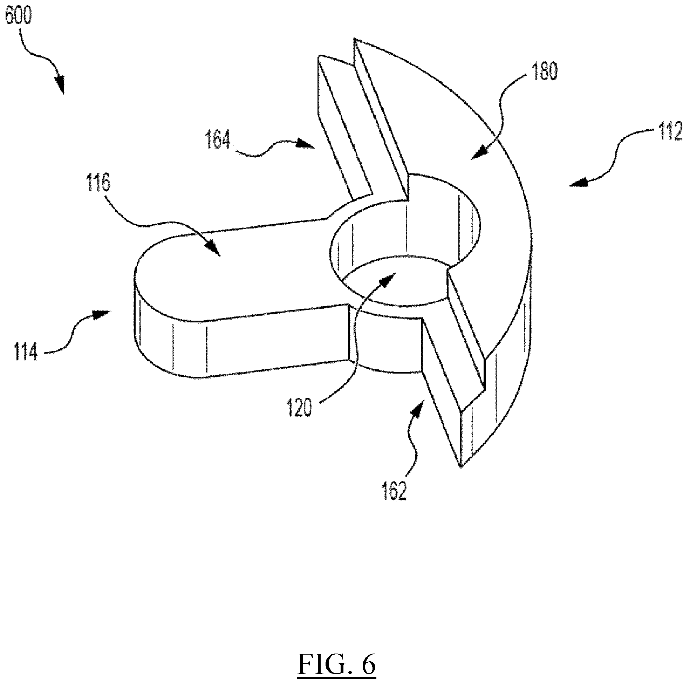

FIG. 6 is a perspective view of another example mechanical barrier 600, in accordance with the disclosed technology. As shown, the mechanical barrier 600 can include a first wing 162 and a second wing 164 similar to the mechanical barrier 500. The mechanical barrier 600, however, can further include one or more ledges 180 that can extend upwardly from the first wing 162 and the second wing 164. The one or more ledges 180 can be configured to align with, and rest against, the anchor member 130. The one or more ledges 180 can extend semi-circumferentially about an axis of the lumen 120. When assembled together, the one or more ledges 180 can help to retain the anchor member 130 within the recess 141 and prevent the anchor member 130 from rotating about an axis defined by the lumen 120.

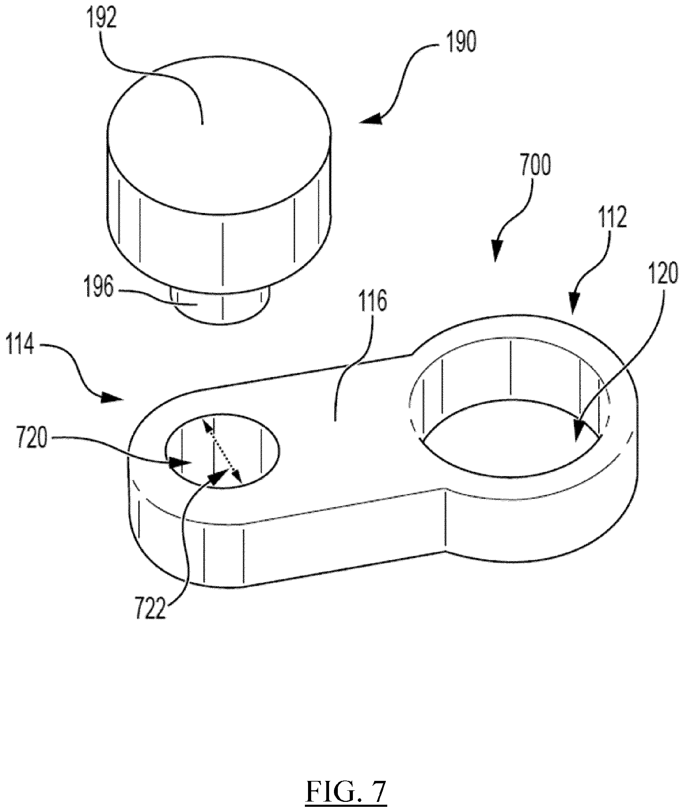

FIG. 7 is a perspective view of yet another example mechanical barrier 700 and a fastener 190, in accordance with the disclosed technology. The mechanical barrier 700 can include some or all of the same features described in relation to the mechanical barriers 100, 500, and 600 previously described herein, however, the mechanical barrier 700 can further include a second lumen 720 extending through the arm 116 near the second end 114. The second lumen 720 can comprise a second lumen diameter 722 that is sized to at least partially receive a fastener 190. The fastener 190 can be configured to extend at least partially into the second lumen 720 to help secure the mechanical barrier 700 to the deflectable tip 140. The fastener 190 can include a circular base 192 and a cylindrical member 196 extending outwardly from the circular base 192. For example, the circular base 192 can be a head of a fastener and the circular member 196 can be an elongated portion of a fastener.

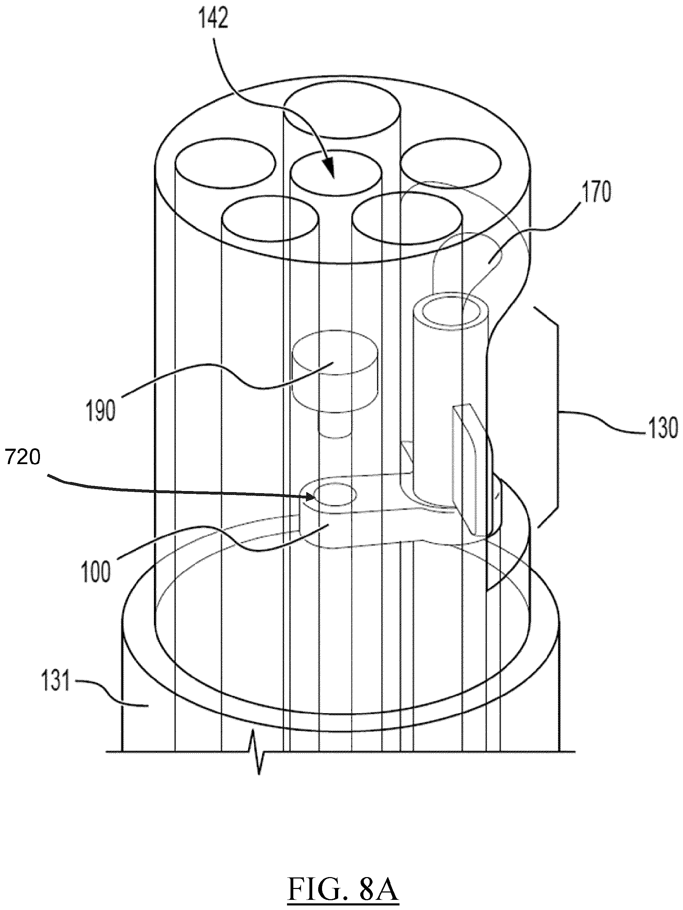

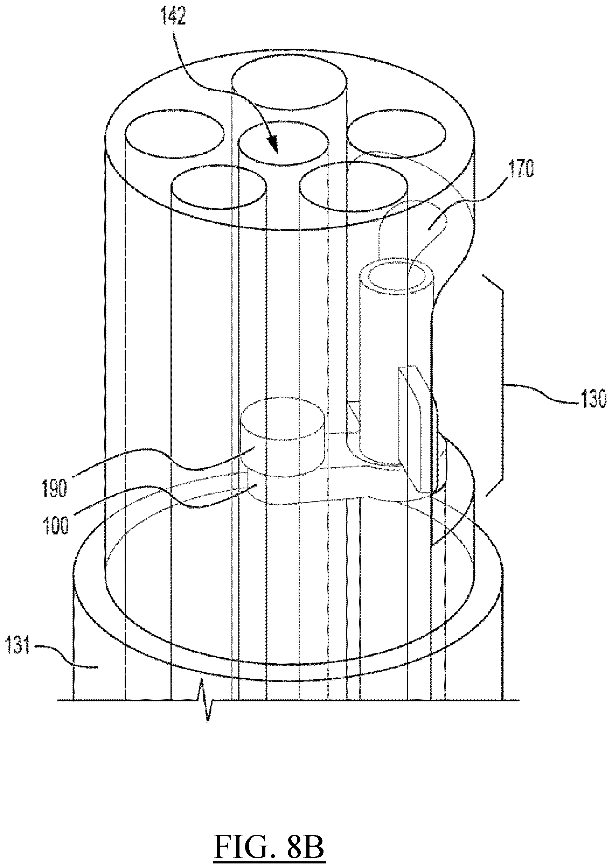

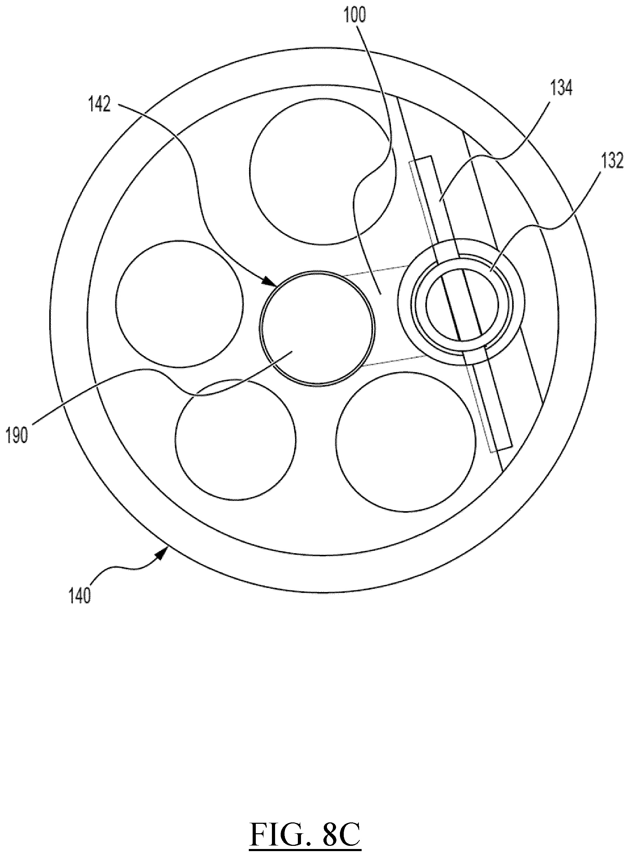

FIG. 8A is a side perspective view of a medical probe comprising an elongated tube 131, an anchor member 130, a mechanical barrier 700, and a fastener 190, in accordance with the disclosed technology. As shown, the mechanical barrier 700 can be assembled with the anchor member 130 and the deflectable tip 140 as previously described with the arm 116 extending into the central lumen 142. The fastener 190 can then be inserted into the central lumen 142 and aligned with the second lumen 720. As further shown in FIG. 8B (which is another side view) and FIG. 8C (which is a top view), the fastener 190 can then be inserted into the second lumen 720. The diameter of the second lumen 720 can be less than the diameter of the first lumen 120. Alternatively, the diameter of the second lumen 720 be the same as, or greater than, the first lumen 120. As will be appreciated, the fastener 190 can be configured to secure the mechanical barrier 700 in place and further help to distribute the force applied to the anchor member 130 toward the center of the deflectable tip 140. The fastener 190 can be secured to the mechanical barrier 700 by a press fit, complementary threaded features, soldering, welding, adhesive, or any other suitable method known in the art.

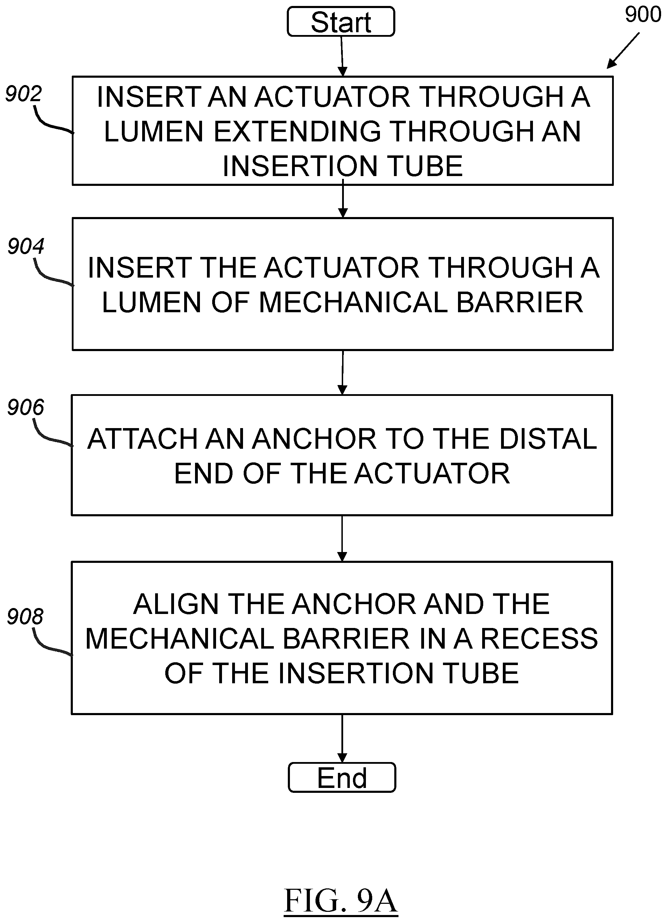

FIG. 9A is a flowchart illustrating a method 900 of manufacturing a medical probe in accordance with the disclosed technology. In some examples, the medical probe disclosed in method 900 is substantially similar to any of the medical probes illustrated and described in relation to FIGS. 2-8C. Method 900 can include inserting 902 an actuator (e.g., a pull wire or a push rod) through a lumen extending through an insertion tube (e.g., elongated tube 131). The method 900 can include inserting 904 the actuator through a lumen (e.g. lumen 120) of a mechanical barrier (e.g., mechanical barrier 100, 500, 600, or 700) and attaching 906 an anchor (e.g., anchor member 130) to the distal end of the actuator. The method 900 can further include aligning 908 the anchor and the mechanical barrier in a recess of the insertion tube.

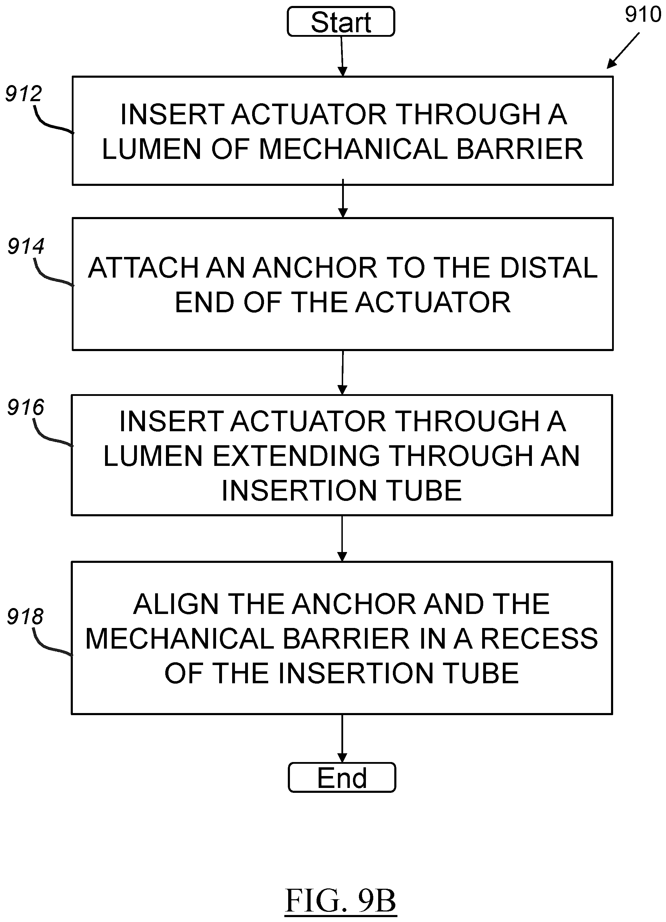

FIG. 9B is a flowchart illustrating another method 910 of manufacturing a medical probe in accordance with the disclosed technology. In some examples, the medical probe disclosed in method 910 is substantially similar to any of the medical probes illustrated and described in relation to FIGS. 2-8C. Method 910 can include inserting 912 an actuator (e.g., a pull wire or a push rod) through a lumen (e.g. lumen 120) of a mechanical barrier (e.g., mechanical barrier 100, 500, 600, or 700) and attaching 914 an anchor (e.g., anchor member 130) to the distal end of the actuator. The method 910 can include inserting 916 the actuator through a lumen extending through an insertion tube (e.g., elongated tube 131). The method 910 can further include aligning 918 the anchor and the mechanical barrier in a recess of the insertion tube.

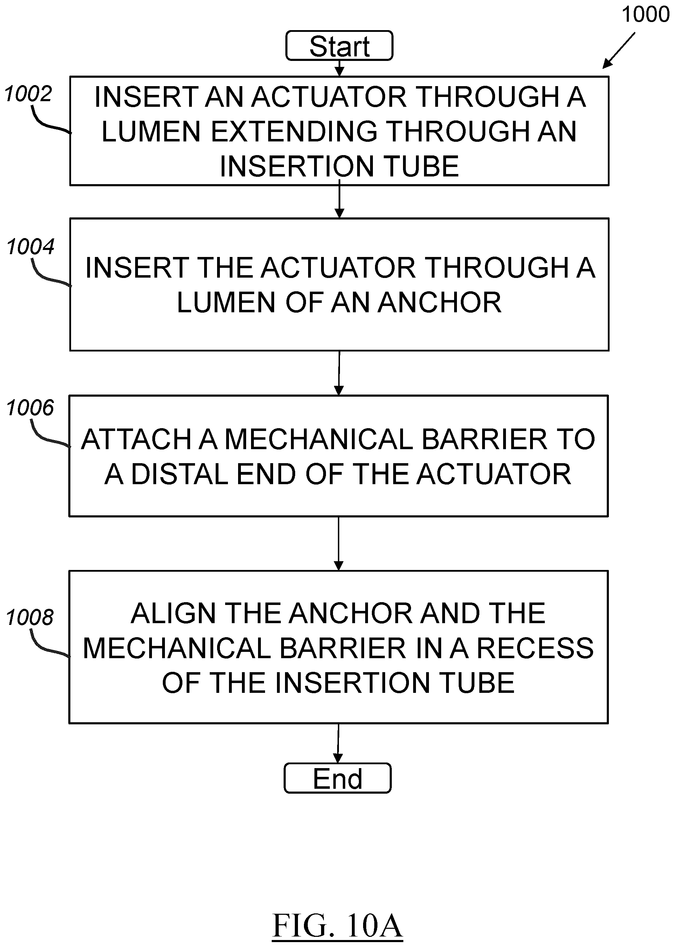

FIG. 10A is a flowchart illustrating another method 1000 of manufacturing a medical probe in accordance with the disclosed technology. In some examples, the medical probe disclosed in method 1000 is substantially similar to any of the medical probes illustrated and described in relation to FIGS. 2-8C. Method 1000 can include inserting 1002 an actuator (e.g., a pull wire or a push rod) through a lumen extending through an insertion tube (e.g., elongated tube 131). The method 1000 can include inserting 1004 the actuator through a lumen of an anchor (e.g., anchor member 130) and attaching 1006 a mechanical barrier (e.g., mechanical barrier 100, 500, 600, or 700) to a distal end of the actuator. The method 1000 can further include aligning 1008 the anchor and the mechanical barrier in a recess of the insertion tube.

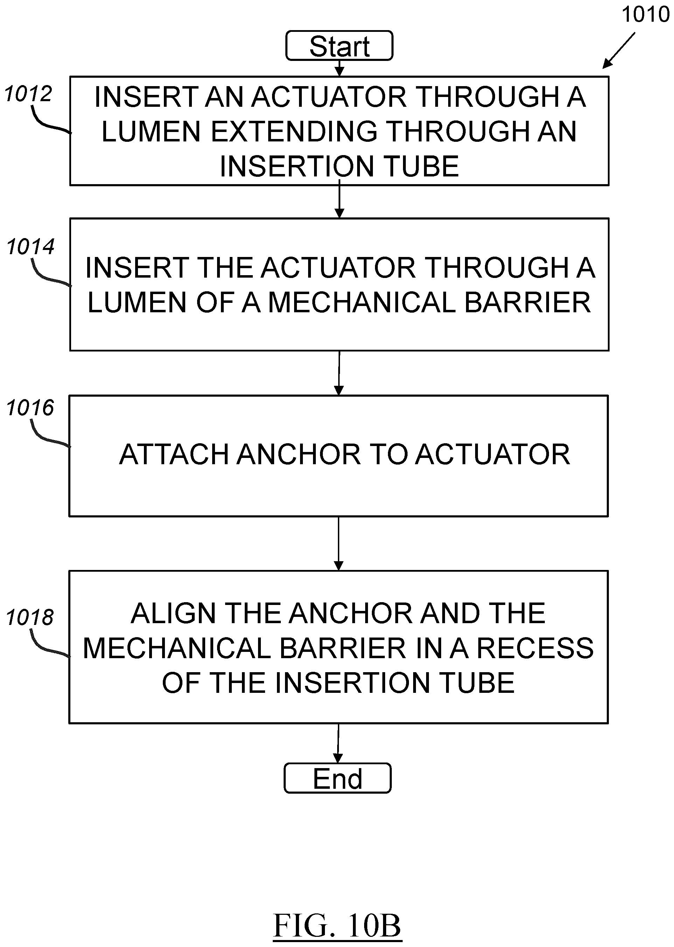

FIG. 10B is a flowchart illustrating another method 1010 of manufacturing a medical probe in accordance with the disclosed technology. In some examples, the medical probe disclosed in method 1010 is substantially similar to any of the medical probes illustrated and described in relation to FIGS. 2-8C. Method 1010 can include inserting 1012 an actuator (e.g., a pull wire or a push rod) through a lumen extending through an insertion tube (e.g., elongated tube 131) and inserting 1014 the actuator through a lumen of a mechanical barrier (e.g., mechanical barrier 100, 500, 600, or 700). The method 1010 can include attaching 1016 anchor to the actuator and aligning 1018 the anchor and the mechanical barrier in a recess of the insertion tube.

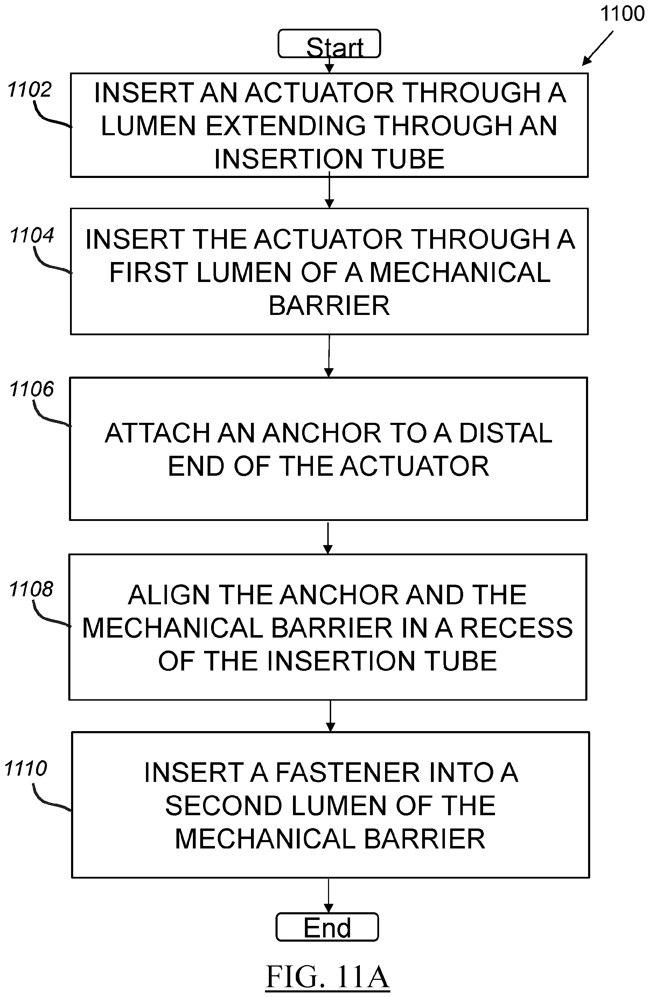

FIG. 11A is a flowchart illustrating yet another method 1100 of manufacturing a medical probe in accordance with the disclosed technology. In some examples, the medical probe disclosed in method 1000 is substantially similar to any of the medical probes illustrated and described in relation to FIGS. 2-8C. Method 1200 can include inserting 1102 an actuator (e.g., a pull wire or a push rod) through a lumen extending through an insertion tube (e.g., elongated tube 131). The method 1100 can include inserting 1104 the actuator through a first lumen (e.g., lumen 120) of a mechanical barrier (e.g., mechanical barrier 100, 500, 600, or 700) and attaching 1106 an anchor (e.g., anchor member 130) to the end of the actuator. The method 1000 can further include aligning 1108 the anchor and the mechanical barrier in a recess of the insertion tube and inserting 1110 a fastener (e.g., fastener 190) into a second lumen (e.g., second lumen 720) of the mechanical barrier, thereby attaching the fastener to the mechanical barrier within the distal tip.

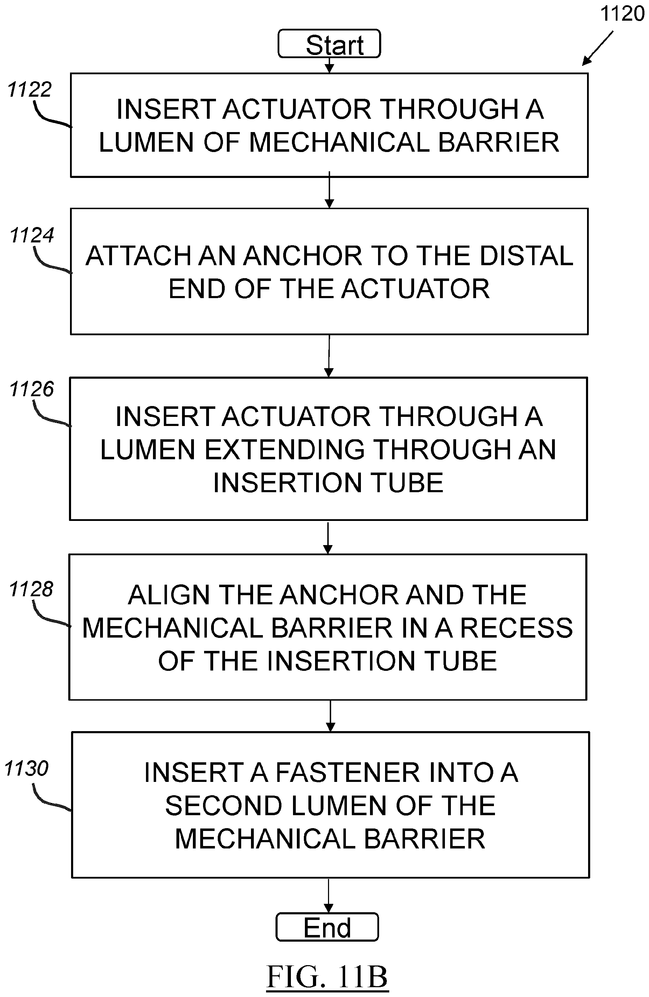

FIG. 11B is a flowchart illustrating yet another method 1120 of manufacturing a medical probe in accordance with the disclosed technology. In some examples, the medical probe disclosed in method 1120 is substantially similar to any of the medical probes illustrated and described in relation to FIGS. 2-8C. Method 1120 can include inserting 1122 an actuator (e.g., a pull wire or a push rod) through a lumen (e.g. lumen 120) of a mechanical barrier (e.g., mechanical barrier 100, 500, 600, or 700) and attaching 1124 an anchor (e.g., anchor member 130) to the distal end of the actuator. The method 1120 can include inserting 1226 the actuator through a lumen extending through an insertion tube (e.g., elongated tube 131). The method 1120 can further include aligning 1128 the anchor and the mechanical barrier in a recess of the insertion tube and inserting 1130 a fastener (e.g., fastener 190) into a second lumen (e.g., second lumen 720) of the mechanical barrier, thereby attaching the fastener to the mechanical barrier within the distal tip.

The methods just described can be used with the devices and features described herein. The methods are offered for illustrative purposes and should not be construed as limited to include only the elements described herein and can include alternative orders of steps and/or intervening steps not described herein.

The disclosed technology described herein can be further understood according to the following clauses:
Clause 1: A medical probe comprising: an elongated tube extending along a longitudinal axis from a proximal end to a distal end; an actuator member extending along the elongated tube from approximately the proximal end to approximately the distal end; an anchor member attached to the actuator member near the distal end of the elongated tube; and a mechanical barrier disposed between the anchor member and the elongated tube, the mechanical barrier comprising a lumen extending through the mechanical barrier along a lumen axis, the lumen configured to at least partially receive the anchor member.
Clause 2: The medical probe of clause 1, the mechanical barrier being disposed proximate the anchor member.
Clause 3: The medical probe of clause 1, the mechanical barrier being disposed distal the anchor member.
Clause 4: The medical probe of clause 1, the anchor member comprising a ferrule extending along a ferrule axis and one or more extensions extending outward from the ferrule perpendicular to the ferrule axis.
Clause 5: The medical probe of clause 1, the actuator member comprising at least one of a puller wire or a rod.
Clause 6: The medical probe of clause 1, the mechanical barrier being configured to prevent the anchor member from damaging the elongated tube.
Clause 7: The medical probe of clause 1, the mechanical barrier further comprising one or more ledges extending outward from the mechanical barrier in a direction along the lumen axis, the one or more ledges configured to help align the anchor member along the elongated tube when assembled together with the mechanical barrier.
Clause 8: The medical probe of clause 7, the one or more ledges extending semi-circumferentially about the lumen axis.
Clause 9: The medical probe of clause 1, the medical probe further comprising: one or more wings extending outward from the mechanical barrier near a first end, the one or more wings configured to fit within a recess defined by the elongated tube.
Clause 10: The medical probe of clause 9, the one or more wings extending outwardly from the mechanical barrier in a semi-circular shape.
Clause 11: The medical probe of clause 10, the one or more wings comprising a first wing and a second wing, the first wing disposed opposite the second wing with respect to the lumen axis.
Clause 12: The medical probe of clause 1, the mechanical barrier further comprising an arm extending outwardly from the mechanical barrier perpendicular to the lumen axis, the arm configured to extend into a lumen defined by the elongated tube.
Clause 13: The medical probe of clause 12, the lumen being a first lumen disposed near a first end of the mechanical barrier, the mechanical barrier further defining a second lumen extending through the arm near a second end of the mechanical barrier opposite the first end, the medical probe further comprising a fastener configured to extend at least partially into the second lumen to secure the mechanical barrier to the elongated tube when assembled with the elongated tube.
Clause 14: The medical probe of clause 13, the fastener comprising: a generally circular base comprising a first diameter; and a generally cylindrical member comprising a second diameter, the first diameter being greater than the second diameter and the generally cylindrical member extending outwardly from the generally circular base.
Clause 15: The medical probe of clause 14, the generally cylindrical member being configured to be disposed at least partially within the second lumen.
Clause 16: The medical probe of clause 14, the generally cylindrical member being further configured to be secured at least partially in the second lumen via at least one of a press fit, complementary threaded features, soldering, welding, and adhesive..
Clause 17: The medical probe of clause 14, the second lumen comprising a second lumen inner diameter, the second lumen inner diameter being less than an inner diameter of the first lumen.
Clause 18: A medical device comprising: an elongated tube extending along a longitudinal axis from a proximal end to a distal end; an actuator member extending along the elongated tube; an anchor member attached to the actuator member near the distal end of the elongated tube, the actuator member and anchor member configured to cause a deflectable tip of the elongated tube to deflect radially outward from the longitudinal axis when actuated; and a mechanical barrier disposed between the anchor member and the elongated tube, the mechanical barrier configured to prevent the anchor member from damaging the elongated tube.
Clause 19: The medical device of clause 18, the mechanical barrier defining: a lumen extending through the mechanical barrier along a lumen axis, the lumen configured to receive at least a portion of the anchor member; and an arm extending outwardly from the mechanical barrier perpendicular to the lumen axis, the arm configured to extend into a lumen defined by the deflectable tip.
Clause 20: The medical device of clause 19, the lumen being a first lumen and the lumen axis being a first lumen axis, the mechanical barrier further defining a second lumen extending through the arm along a second lumen axis, the mechanical barrier further comprising a fastener configured to extend at least partially into the second lumen to secure the mechanical barrier to the deflectable tip when assembled with the deflectable tip.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A medical probe comprising:
an elongated tube extending along a longitudinal axis from a proximal end to a distal end;
an actuator member extending along the elongated tube from approximately the proximal end to approximately the distal end;
an anchor member attached to the actuator member near the distal end of the elongated tube; and
a mechanical barrier disposed between the anchor member and the elongated tube, the mechanical barrier comprising a lumen extending through the mechanical barrier along a lumen axis, the lumen configured to at least partially receive the anchor member.

2. The medical probe of claim 1, the mechanical barrier being disposed i) proximate the anchor member, or ii) distal the anchor member.

3. The medical probe of claim 1, the anchor member comprising a ferrule extending along a ferrule axis and one or more extensions extending outward from the ferrule perpendicular to the ferrule axis.

4. The medical probe of claim 1, the actuator member comprising at least one of a puller wire or a rod.

5. The medical probe of claim 1, the mechanical barrier being configured to prevent the anchor member from damaging the elongated tube.

6. The medical probe of claim 1, the mechanical barrier further comprising one or more ledges extending outward from the mechanical barrier in a direction along the lumen axis, the one or more ledges configured to help align the anchor member along the elongated tube when assembled together with the mechanical barrier, optionally the one or more ledges extending semi-circumferentially about the lumen axis.

7. The medical probe of claim 1, the medical probe further comprising:
one or more wings extending outward from the mechanical barrier near a first end, the one or more wings configured to fit within a recess defined by the elongated tube.

8. The medical probe of claim 7, the one or more wings extending outwardly from the mechanical barrier in a semi-circular shape, optionally the one or more wings comprising a first wing and a second wing, the first wing disposed opposite the second wing with respect to the lumen axis.

9. The medical probe of claim 1, the mechanical barrier further comprising an arm extending outwardly from the mechanical barrier perpendicular to the lumen axis, the arm configured to extend into a lumen defined by the elongated tube.

10. The medical probe of claim 9, the lumen being a first lumen disposed near a first end of the mechanical barrier, the mechanical barrier further defining a second lumen extending through the arm near a second end of the mechanical barrier opposite the first end, the medical probe further comprising a fastener configured to extend at least partially into the second lumen to secure the mechanical barrier to the elongated tube when assembled with the elongated tube.

11. The medical probe of claim 10, the fastener comprising:
a generally circular base comprising a first diameter; and
a generally cylindrical member comprising a second diameter, the first diameter being greater than the second diameter and the generally cylindrical member extending outwardly from the generally circular base.

12. The medical probe of claim 11, the generally cylindrical member i) being configured to be disposed at least partially within the second lumen, or being further configured to be secured at least partially in the second lumen via at least one of a press fit, complementary threaded features, soldering, welding, and adhesive.

13. The medical probe of claim 11, the second lumen comprising a second lumen inner diameter, the second lumen inner diameter being less than an inner diameter of the first lumen.

14. A medical device comprising:
an elongated tube extending along a longitudinal axis from a proximal end to a distal end;
an actuator member extending along the elongated tube;
an anchor member attached to the actuator member near the distal end of the elongated tube, the actuator member and anchor member configured to cause a deflectable tip of the elongated tube to deflect radially outward from the longitudinal axis when actuated; and
a mechanical barrier disposed between the anchor member and the elongated tube, the mechanical barrier configured to prevent the anchor member from damaging the elongated tube.

15. The medical device of claim 14, the mechanical barrier defining:
a lumen extending through the mechanical barrier along a lumen axis, the lumen configured to receive at least a portion of the anchor member; and
an arm extending outwardly from the mechanical barrier perpendicular to the lumen axis, the arm configured to extend into a lumen defined by the deflectable tip, optionally the lumen being a first lumen and the lumen axis being a first lumen axis, the mechanical barrier further defining a second lumen extending through the arm along a second lumen axis, the mechanical barrier further comprising a fastener configured to extend at least partially into the second lumen to secure the mechanical barrier to the deflectable tip when assembled with the deflectable tip.
